# EUROPEAN PATENT SPECIFICATION

(11) **EP 3 836 934 B1**
(45) Date of publication and mention of the grant of the patent: **21.06.2023**
(21) Application number: 18930471.0
(22) Date of filing: 17.08.2018
(51) Int. Cl.: A61K 31/522, A61K 31/573, A61K 9/06, A61K 47/14, A61K 47/10, A61K 45/06

(54) **PHASE STABLE TOPICAL COMPOSITION COMPRISING ACYCLOVIR AND HYDROCORTISONE**
PHASENSTABILE TOPISCHE ZUSAMMENSETZUNG MIT ACYCLOVIR UND HYDROCORTISON
COMPOSITION TOPIQUE À PHASES STABLES COMPRENANT DE L'ACYCLOVIR ET DE L'HYDROCORTISONE

(43) Date of publication of application: 23.06.2021
(73) Proprietor: Ilko Ilaç Sanayi Ve Ticaret Anonim Sirketi, Sancaktepe/Istanbul (TR)
(72) Inventor: ÖNCEL, Hatice, Sancaktepe/Istanbul (TR); ÇAPAN, Yilmaz, Çankaya/Ankara (TR); PINARBASLI, Onur, Çankaya/Ankara (TR); BULUT, Burcu, Çankaya/Ankara (TR); SARRAÇOGLU, Nagehan, Çankaya/Ankara (TR)
(86) International application number: PCT/TR2018/050438
(87) International publication number: WO 2020/036545

(56) References cited:
- WO-A1-00/29027
- WO-A1-96/24355
- CN-A- 103 948 605

## Description

### Technical field of the Invention:

This invention relates to a stableantiviral topical composition suitable for orofacial or genital application.Stable topical composition comprisesa combination of an antiviral substance which is acyclovir and an anti-inflammatory glucocorticoid which is hydrocortisone;which exhibit no phase separation at a temperature of about 40°C±2°C and relative humidity of about 75%±5% for a period of at least 3 months.

### Background of the Invention:

Acyclovir,2-amino-1,9-dihydro-9-[(2-hydroxyethoxy)methyl]-6H-purin-6-one, is a synthetic nucleoside analogue active against herpes viruses. The maximum solubility of acyclovir in water at 37°C is 2.5 mg/mL. The pKa's of acyclovir are 2.27 and 9.25. Its empirical formula is C₈H₁₁N₅O₃andthe structural formula is provided in Figure 1:

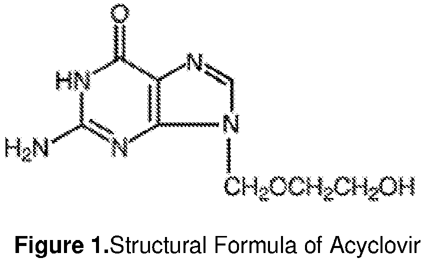

Hydrocortisone, pregn-4-ene-3,20-dione,11,17,21-trihydroxy-(11(beta))-, is an anti-inflammatory corticosteroid. Its empirical formula is C₂₁H₃₀O₅and the structural formula is provided in Figure 2:

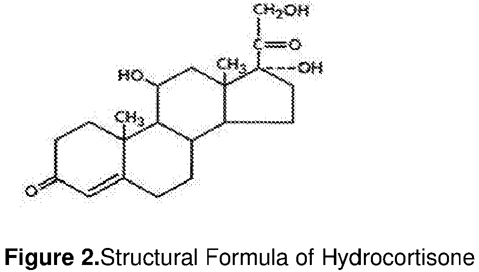

The herpes simplex virus, also known as HSV, is an infection that causes herpes. Herpes can appear in various parts of the body, most commonly on the genitals or mouth. There are two types of the herpes simplex virus. Type 1 is also known as oral herpes; this type can cause cold sores and fever blisters around the mouth and on the face. Type 2 is generally responsible for genital herpes outbreaks. Guanosine nucleoside analogues such as acyclovir, penciclovir or omaciclovir are efficacious against various herpes viruses, such as herpes simplex type 1 or 2 in cell culture experiments. In this present invention, acyclovir is preferred with its low molecular weight, low toxicity and inexpensiveness properties.

The anti-herpes virus activity of acyclovir has been demonstrated in inhibiting the replication of herpes simplex virus in tissue culture cells (O'Brien, W., et al., Antimicrob. Agents and Chemother. 34:1178-1182 (1990); it has also been demonstrated in clinical studies wherein patients infected with HSV were treated with orally administered acyclovir (Whitley, R., Immunobiol. and Prophylaxis of Human Herpesvirus Infections, C. Lopez et al, (eds) Plenum Press, NY 1990; and Straus, S., Sexually Transmitted Diseases 16(2):107-113 (1989).

Acyclovir is the treatment of choice for mucosal and cutaneous HSV infections, although patients receiving topical acyclovir therapy experience some reductions of their symptoms, healing is slow and incomplete (Spruance, S., et al., J. Infect. Dis. 146:85-90 (1982); and Spruance, S., et al., Antimicrob. Agents Chemother. 25:553-555 (1984)). Combination treatment using acyclovir together with interferon for herpes virus infected cultured cells (O'Brien, W., et al., Antimicrob. Agents and Chemother. 34(6):1178-1182 (1990)) or using acyclovir together with A1110U, an HSV inactivator, as a topical therapy for herpetic keratitis in athymic mice (Lobe, D., et al., Antiviral Research 15:87-100 (1991)) showed synergistic anti-herpes virus I activity over the use of acyclovir alone.

An alternative approach to enhancing the efficacy of topical guanosine antivirals is described in Evans et al (2002 Antimicrob. Agents Chemother. 46(6) 1870-1874). This reference describes a phase II clinical trial in a UV induced protocol, employing an antiviral/immunomodulatory combination of 5% acyclovir and 1% hydrocortisone. Healing time was reduced by 1.1 days (P:0.04) and there was a trend toward a reduction in maximum lesion size (P:0.07).

Therefore, it could be of advantage to combine the antiviral with an adjuvant or other additive in order to increase the effect on the herpes virus to be treated, for instance in cases of resistance developed to the antiviral substance.

The cream dosage form of acyclovir and hydrocortisone are commercially marketed under the trade name of Zovirax Duo^{®} (GSK) (Acyclovir and Hydrocortisone 50 mg/g - 10 mg/g).

The combination of acyclovir and hydrocortisone composition are useful for the treatment or prophylaxis of diseases caused by members of the herpesvirus family, such as herpes simplex type 1 (predominantly an orofacial infection), herpes simplex type 2 (predominantly a genitoanal infection), varicella Zoster virus primary infection (chicken pox) and secondary infection (shingles), human herpesvirus type 6 and 8 (implicated in the skin condition Kaposi's sarcoma) and the like.

EP0809498 B1 (Medivir AB) describes pharmaceutical compositions comprising a topically acceptable antiviral agent and an anti-inflammatory glucocorticoid in a pharmaceutically acceptable carrier that can be preferably used for the treatment of recurrent herpes virus infections. The carriers disclosed in this patent comprise conventional formulation for each of the respective active agents, which is a conventional antiviral formulation, is combined with a conventional glucocorticoid formulation. Such combined formulations have a large number of ingredients having potential for interactingwith each other, leading to unknown stability and physicochemical characteristics.

Co-formulation of guanosine antivirals such as acyclovir (typically hydrophilic) with glucocorticoids such as hydrocortisone (typically lipophilic) is not straightforward in view of the very different physicochemical properties of the active substances. Short shelf life, unstable emulsions and crystal growth are frequent difficulties when conventional acyclovir formulations are used in combination with a glucocorticoid.

EP1131104 B1 (Medivir AB) describes topical antiviral formulations in the form of an oil in water emulsion comprising 0.1-10% (w/w) hydrocortisone and 1-7% (w/w) of an antiviral nucleoside analogue selected from acyclovir, penciclovir or 9-(4-hydroxy-2-(hydroxymethyl)butyl)guanine in a pharmaceutical carrier comprising propylene glycol and an isopropyl C₁₂-C₂₂ alkanoic acid ester, characterized in that the carrier comprises, relative to the total weight of the composition, 15-25% (w/w) propylene glycol and 10-25% (w/w) of an isopropyl C₁₂-C₂₂ alkanoic acid ester.The reference product (Zovirax Duo^{®} (GSK)) which is formulated with 20% (w/w) propylene glycol, 15% (w/w) of isopropyl myristate and 6.75% (w/w) cetostearyl alcohol (the formulation is given at EP1131104B1) has phase separation problem especially at accelerated condition (40°C±2°C, 75%RH±5%).

The superiority of the present invention with the formulations described in the previous patent is the elimination of phase separation problems of the topical formulationat a temperature of about 40°C±2°C and relative humidity of about 75%±5% for a period of at least 3 monthswith optimizing the amount of used excipients while having same in vitro release which is shown by in vitro release testing for topical cream formulations. Acyclovir and hydrocortisone are often challenging to formulate as they exhibit chemical instability and complex physical instabilities, especially in topical form. Both chemical and physical stability are critical to an effective and commercial pharmaceutical formulation. An unstable formulation results in loss of potency, poor quality and patient incompliance. Therefore, topical formulations, especially, must remain stable in the container while stored long term and then when applied to the skin. Indeed, combination of acyclovir and hydrocortisone cream products have some stability problems at shelf life. Especially there is an unstable emulsion which shows phase separation at accelerated stability condition. Phase separation condition is occurred when an emulsion breaks the oily and aqueous phases separate, leading to unwanted coalescence, i.e. a kind of confluence or coagulation of the constituents. Therefore, there remains a need for a dosage form for the topical administration of acyclovir and hydrocortisone, which provides effective antiviral treatment, and which remains phase stable over a long storage term. It is the object of this invention to formulate a pharmaceutical topical composition comprising acyclovir and hydrocortisone and the composition does not phase separate.

### Detailed Description of the Invention:

An object of the present invention is to formulate a phase stable pharmaceutical topical composition at a temperature of about 40°C±2°C witha relative humidity of about 75%±5% for a period of at least 3 months. Mentioned phase stable pharmaceutical composition comprisesa therapeutically effective amount of an antiviral substance which is acyclovir and an anti-inflammatory glucocorticoid, preferably hydrocortisone. The composition comprises 7.5-12.5% (w/w) of isopropyl myristate, and 10-15% (w/w) of cetostearyl alcohol. Anti-inflammatory glucocorticoids suitable for the purpose of the present invention can be a naturally occurring or a synthetic topical glucocorticoid that is glucocorticosteroid.

The glucocorticoid can be selected from any of the Group I-III glucocorticoids, according to a classification system for topical glucocorticoids used in the Nordic countries, corresponding to less potent, low or moderately potent glucocorticoids. Examples of glucocorticosteroids are hydrocortisone, alclometasone, desonide, fluprednidene, flumethasone, hydrocortisone butyrate, clobetasone, triamcinolone acetonide, betamethasone, budesonide, desoximethasone, diflorosane, fluocinolone, fluocortolone, fluticasone, methylprednisolone aceponate, mometasone and rofleponide. Hydrocortisone and its esters are particularly preferred in this invention.

Creams and emulsions are the most diverse and widely used dosage forms for topical dermatological applications. Creams are semisolid dosage forms that contain one or more drug substances dissolved or dispersed in a suitable base. They are two-phase systems typically containing hydrophilic ingredient in an aqueous phase and lipophilic ingredients in an oil phase. The two-phase system term traditionally has been applied to semisolids that possess a relatively soft, spreadable consistency formulated as either water-in-oil or oil-in-water emulsions. They are two-phase systems typically containing hydrophilic ingredient in an aqueous phase and lipophilic ingredients in an oil phase. In order for topical emulsions to function effectively they require certain functional excipients. Therefore, selection of excipients and determination of the amount to be used are critical especially in two phase topical formulation in order to prevent phase separation. Consequently, this composition has been developed in order to eliminate the stability problems, especially phase separation, with respect to traditional antiviral products.

The term "therapeutically effective amount" refers to an amount,which achieves a desired effect, when administered to a living subject. The relative amount of the antiviral substance in a pharmaceutical composition according to the present invention can be within the range of 0.1-10% (w/w), preferably 5% (w/w) acyclovir. The anti-inflammatory glucocorticoidconcentration can be within the range of 0.005-3% (w/w), preferably 1% (w/w).

The term "topical composition" (synonymously, "topical formulation") is used herein to refer to a pharmaceutical preparation intended for topical or local application to an afflicted region of a subject in need thereof, and includes such dosage forms as gel, cream, ointment, emulsion, suspension, solution, drops, lotion, spray or foam. Preferably, in the present invention the topical formulation is in the form of a cream.

As used herein, the term "water soluble" means water soluble or water dispersible. A water soluble compound has a water solubility of at least 0.1 g (grams) per 100 ml (milliliters) of water and forms a true solution. A water soluble compound can be inherently water soluble or can be made water soluble by the addition of a solubilizing compound, such as a coupling agent, a co-surfactant, or a solvent.

In this invention, weight percentages (w/w) refer to the weight of the component relative to weight of the composition.

As used herein, "stable" refers to a composition that substantial color change, turbidity or phase separation can be seen with naked eye easily. No phase separation should be observed in either aqueous and/or non-aqueous components for at least about 3 months at 40°C±2°C, 75%RH±5%. More preferably, no phase separation should be observed in either aqueous and/or non-aqueous components for at least about 6 months at 40°C±2°C, 75%RH±5%.

A composition comprising acyclovir and hydrocortisone in cream form is difficult due to very different physicochemical properties of the actives. Acyclovir suffers from the disadvantage that it has low solubility in water and is almost totally insoluble in hydrophobic solvent systems.

A combination product composition comprising a strongly lipophilic component such as hydrocortisone and a moderately lipophobic component such as an acyclic nucleoside analogue is difficult in the case of conventional pharmaceuticals, but is even more difficult in the case of a topical preparation where stability problems are involved.

Phase separation is a problem that can be encountered over time in topical forms when there is a tendency for phase decomposition. Thus, whilst the manufacturing process results in a homogeneous emulsion, if the product is stored for any length of time before being used, it may start to separate into aqueous and non-aqueous phases and present a "curdled" (volume of clear serum topped by a distinct "creamy" phase) product to the consumer. In general, this phenomenon may create an adverse impression in the minds of some consumers regarding the product's quality and acceptability. In the present invention, the phase separation problem after storage at about 40°C±2°C with 75%±5% relative humidity is solved by using 10-15% (w/w) of cetostearyl alcohol in conjunction with 7.5-12.5% (w/w) isopropyl myristate in a cream composition of acyclovir and hydrocortisone.

The compositions of the invention arebiphasic and comprise discrete oil and aqueous phases, either as oil in water or water in oil emulsion. Components of the oil phase may include conventional fats and oils and their esters, as found in the Europeanand other pharmacopoeias. Oil phase components are preferably non-greasy, non-staining and washable. Conventionalpharmaceutical oil phase components include mineral oils such as vaseline, liquid paraffin and the like, alkanoic acidssuch as stearic acid and fatty alcohols such as cetostearylalcohol, straight or branched chain mono or dibasic alkylesters such as di-isopropyl adipate, isocetyl stearate, propylene glycol diester of coconut fatty acids, decyloleate, butylstearate, 2-ethylhexyl palmitate and other 2-ethylhexanoicacid esters and the like. The composition of the invention comprises 10-15% (w/w) of cetostearyl alcohol. In topical pharmaceutical compositions, cetostearyl alcohol increases the viscosity and act as an emulsifier in both water-in-oil and oil-in-water emulsions. Also, it stabilizes an emulsion and acts as a co-emulsifier, thus decreasing the total amount of surfactant required to form a stable emulsion.

To achieve a consistent supply of therapeutic active ingredient at the site of action during the treatment of skin diseases, it has been found that the use of penetration enhancer is essential. However, the uses of some penetration enhancers are problematic. For example, the use of dimethyl sulfoxide causes a foul taste and body odor, burning and erythema on the skin, a reduction in the relucency of the lens cortex and tissue necrosis in animals.(Martindale, The Extra Pharmacopoeia, pages 1461 - 1463, Twenty-Seventh Edition, 1977). Dimethyl formamide and dimethylacetamide also cause a sensation of burning and erythema on the skin. As a result, there exists a need for a novel agent that enhances the absorption of therapeutic agents through the skin and is substantially devoid of the disadvantages and drawbacks that to date have characterized many prior art penetration enhancing agents. Thus, penetration enhancers like isopropyl alkanoic acid esters include the dodecanate, myristate, palmitate, stearate, eicosanate or behenoate esters are used to overcome these problems. The composition of the invention comprises 7.5-12.5% (w/w) of isopropyl myristate. Isopropyl myristate is widely used in cosmetics and topical pharmaceutical formulations as a penetration enhancer and is generally regarded as a non-toxic and non-irritant material.

The composition of the invention comprises about 20% (w/w)of propylene glycol. Conveniently 20% (w/w)of propylene glycol content generally assures a good preservative effect without needing exogenous preservatives in the composition.

The compositions of the invention can also include conventional excipients such as surfactants, surface anesthetics, sunscreens, flavors, scents, emollients or skin tone colorants and masks.

The European Pharmacopoeia describes a number of pharmaceutically acceptable emulsifiers (surfactant) including anionic,cationic and non-ionic emulsifiers. Exemplary non-ionic emulsifiersinclude cetomacrogols,such as cetomacrogol 1000, ethylene ordiethylene glycol monostearate, glyceryl esters such asthebehenate, oleate, stearate etc, laurethcompoundssuch as lauromacrogols, macrogol monomethyl ethers,mono- and diglycerides, nonoxinols, octoxinols,poloxamers such as poloxamer 407, polyoxylcastoroils, polyoxyl stearates, polysorbates, polyvinyl alcohols,propylene glycol diacetates, sorbitan esters andthe like. In this invention, poloxamer 188 is preferred as non-ionic surfactant.Exemplary anionic emulsifiers include aluminiummonostearate, calcium stearate, sulphatedcastoroil, magnesium stearate, pendecamaine, sodiumoleate, sodium stearate, sodium stearyl fumarate, sodiumtetradecylsulphate, zinc stearate and the like. In this invention, sodium lauryl sulphate is preferred as anionic surfactant.

The following examples are for the purpose of illustration of the invention only and are not intended in any way to limit the scope of the present invention.

### Example 1.Production method of Acyclovir - Hydrocortisone Cream Composition

| | **Amount % (w/w)** |
|---|---|
| Acyclovir | 5.00 |
| Hydrocortisone | 1.00 |
| Cetostearyl Alcohol | 10.0-15.0 |
| Vaseline | 10.00 |
| Liquid Paraffin | 5.65 |
| Isopropyl Myristate | 7.50-15.00 |
| Propylene Glycol | 20.00 |
| Sodium Lauryl Sulphate | 0.80 |
| Poloxamer 188 | 1.00 |
| pH adjusting solution | q.s. |
| Pure water | q.s. |

The oil phase components of cetostearyl alcohol, vaseline, isopropyl myristate and liquid paraffin are added into mixing vessel. On the other side, aqueous phase components of propylene glycol, sodium lauryl sulphate, poloxamer 188 and pure water are added to another mixing vessel. Both vessels are heated to 70°C under agitation. When the phases are at an identical temperature, the oil phase is poured onto the aqueous phase from above while continuing to agitate for 3-5 minutes at the highest possible speed which avoids drawing air into the mixture. The emulsified mixture is then homogenized and cooled with continued agitation to 32-25°C. The active ingredients are added and agitation continued until the active ingredients are wetted and blended in. The pH is controlled and adjusted to pH 4.5-6.5 ± 0.3 with using proper pH adjusting solution. The mixture is once again homogenized and cooled until the cream thickens, around 30°C, before packaging.

### Example 2. Compositions tested for determining the amount of cetostearyl alcohol with using 7.50% isopropyl myristate

| | **Amount % (w/w)** | | |
|---|---|---|---|
| | **Test-1** | **Test-2** | **Test-3** |
| Acyclovir | | 5.00 | |
| Hydrocortisone | | 1.00 | |
| Cetostearyl Alcohol | 10.0 | 12.5 | 15.0 |
| Vaseline | | 10.00 | |
| Liquid Paraffin | | 5.65 | |
| Isopropyl Myristate | | 7.50 | |
| Propylene Glycol | | 20.00 | |
| Sodium Lauryl Sulphate | | 0.80 | |
| Poloxamer 188 | | 1.00 | |
| pH adjusting solution | | q.s. | |
| Pure water | | q.s. | |

The compositions of Example 2 (Test 1-3) were made according to the procedure set out in Example 1 above.

### Example 3. Compositions tested for determining the amount of cetostearyl alcohol with using 10.00% isopropyl myristate

| | **Amount % (w/w)** | | |
|---|---|---|---|
| | **Test-4** | **Test-5** | **Test-6** |
| Acyclovir | | 5.00 | |
| Hydrocortisone | | 1.00 | |
| Cetostearyl Alcohol | 10.0 | 12.5 | 15.0 |
| Vaseline | | 10.00 | |
| Liquid Paraffin | | 5.65 | |
| Isopropyl Myristate | | 10.00 | |
| Propylene Glycol | | 20.00 | |
| Sodium Lauryl Sulphate | | 0.80 | |
| Poloxamer 188 | | 1.00 | |
| pH adjusting solution | | q.s. | |
| Pure water | | q.s. | |

The compositions of Example 3(Test 4-6) were made according to the procedure set out in Example 1 above.

### Example 4.Compositions tested for determining the amount of cetostearyl alcohol with using 12.50% isopropyl myristate

| | **Amount % (w/w)** | | |
|---|---|---|---|
| | **Test-7** | **Test-8** | **Test-9** |
| Acyclovir | | 5.00 | |
| Hydrocortisone | | 1.00 | |
| Cetostearyl Alcohol | 10.0 | 12.5 | 15.0 |
| Vaseline | | 10.00 | |
| Liquid Paraffin | | 5.65 | |
| Isopropyl Myristate | 12.50 | | |
| Propylene Glycol | 20.00 | | |
| Sodium Lauryl Sulphate | 0.80 | | |
| Poloxamer 188 | 1.00 | | |
| pH adjusting solution | q.s. | | |
| Pure water | q.s. | | |

The compositions of Example 4(Test 7-9) were made according to the procedure set out in Example 1 above.

### Example 5.Compositions tested for amount of cetostearyl alcohol with using 15.00% isopropyl myristate

| | **Amount % (w/w)** | | |
|---|---|---|---|
| | **Test-10** | **Test-11** | **Test-12** |
| Acyclovir | | 5.00 | |
| Hydrocortisone | | 1.00 | |
| Cetostearyl Alcohol | 10.0 | 12.5 | 15.0 |
| Vaseline | | 10.00 | |
| Liquid Paraffin | | 5.65 | |
| Isopropyl Myristate | | 15.00 | |
| Propylene Glycol | | 20.00 | |
| Sodium Lauryl Sulphate | | 0.80 | |
| Poloxamer 188 | | 1.00 | |
| pH adjusting solution | | q.s. | |
| Pure water | | q.s. | |

The compositions of Example 5(Test 10-12) were made according to the procedure set out in Example 1 above.

### In Vitro Release Testing

For a topical medicament to be effective it must be readily released from the vehicle matrix and interact intimately with the skin to be treated. On this basis candidate compositions can be ranked based on in vitro release rates through artificial or post mortem skin membranes. This is routinely undertaken using the Franz Diffusion Cell methodology. The rate and extent to which the drug substance is released from the product matrix are particularly relevant to the prediction of relative efficacy of candidate formulations.

In Vitro Release Testing (IVRT) is a useful test to assess product "sameness" under certain scale and post approval changes for semisolid products. The FDA Guidance on Scale up and Post Approval Changes for Semisolid (SUPAC-SS) describes suitable conditions for this testing. The apparatus used for IVRT is a Franz diffusion cell system. It consists of six individual cells. Each cell has a standard open cap ground glass surface with 15 mm diameter orifices, 35 mL volume capacity, and total diameter of 25 mm. About 300 mg of the semisolid preparation is placed uniformly on a synthetic membrane and kept occluded to prevent solvent evaporation and compositional changes. Multiple sampling times (at least 5 times) over an appropriate time period are suggested in order to generate an adequate release profile and to determine the drug release rate. Each aliquot removed is then typically analyzed, for example, by high performance liquid chromatography (i.e., HPLC). After each aliquot was removed, the cell was refilled with a volume of fresh medium equal to the volume of the aliquot that was removed.

The conditions used for IVRT for the example compositions of the invention are as follows:

| | |
|---|---|
| **Receptor medium** | Saline solution (0.9% NaCl solution) |
| **Speed** | 650 rpm |
| **Membrane** | Nylon membrane |
| **Dosage** | 300 mg ± 30 mg |
| **Temperature** | 32°C ± 0.5°C |

Comparative in vitro release tests were conducted based on the above conditions. The test products (Test 1-12) comprising acyclovir and hydrocortisone were tested in vitro and they were compared with a commercial reference product (Zovirax Duo^{®}, GSK; Acyclovir and Hydrocortisone 50 mg/g - 10 mg/g; C79052).

As a result of in vitro release tests made, it was found that the reference product and Test products (Test 1-12) have similar release profiles according to the graphs obtained from the amounts of acyclovir and hydrocortisone passing per unit cm².

### Stability Studies

In order to examine the physical and chemical stability of the products, stability test was assessed under accelerated conditions (40°C±2°C, 75% Relative humidity, RH±5%) for Test products (Test 1-12) and commercially available reference product (Zovirax Duo^{®}, GSK; Acyclovir and Hydrocortisone 50 mg/g - 10 mg/g; C79052).

Typical stability parameters are the homogeneity of the formulation, the absence of coalescence of the emulsion droplets (no "coagulation"), a practically constant viscosity, semisolid structures, and no subsequent crystallization of the active ingredient out of the emulsion.

A serious phase separation was occurred in reference product (Zovirax Duo^{®}, GSK)and in the compositions of Example 5 (Test 10-12) after storage for 3 months at accelerated condition (40°C±2°C, 75%RH±5%). On the other hand no phase separation was occurred in the compositions of Example 2 (Test 1-3), Example 3 (Test 4-6) and Example 4(Test 7-9)after storage for 6 months at accelerated condition (40°C±2°C, 75%RH±5%). Phase separation results inhomogeneous and unuseable cream compositions.

The topical administration of the pharmaceutical compositions of the present invention (Test 1-9) wherein the compositions comprise 7.5-12.5% (w/w) of isopropyl alkanoic acid ester and 10.0-15.0% (w/w) of cetostearyl alcohol have resulted an appropriate penetration and release of the antiviral component, while at the same time avoiding the instability, especiallyphase separation, against the conventional antiviral composition (Zovirax Duo^{®}, GSK) and Example 5.

While reference product has phase separation at accelerated condition (40°C±2°C, 75% RH±5%) after 3 months, the composition prepared in the present invention has no phase separation after storage at about 40°C±2°C and about 75%±5% relative humidity for 6 months.

Marketed product (Zovirax Duo^{®}, GSK) comprise 15% (w/w) of isopropyl myristate and 6.75% (w/w) cetostearyl alcohol and it has a serious phase separation occurred under accelerated conditions (40°C±2°C, 75%RH±5% Relative humidity) with resulting inhomogeneous and unusable cream compositions. Inventors surprisingly discovered that a pharmaceutical topical composition having improved stability, especially phase stability, can be prepared by incorporation of light 7.5-12.5% (w/w) of isopropyl alkanoic acid ester, preferably isopropyl myristate, and 10.0-15.0% (w/w) of cetostearyl alcohol in the composition.

## Claims

1. A phase stable pharmaceutical topical composition comprising a therapeutically effective amount of an antiviral substance which is acyclovir and a therapeutically effective amount of an anti-inflammatory glucocorticoid which is hydrocortisone, wherein the composition comprises 10.0-15.0% (w/w) of cetostearyl alcohol and 7.5-12.5% (w/w) of isopropyl myristate.

2. Pharmaceutical composition according to claim 1, wherein the composition comprises 20% (w/w) propylene glycol.

3. Pharmaceutical composition according to claim 1, wherein the composition comprises 0.1-10% (w/w) acyclovir.

4. Pharmaceutical composition according to claim 3, wherein the composition comprises 5% (w/w) acyclovir.

5. Pharmaceutical composition according to claim 1, wherein the composition comprises 0.005-3% (w/w) hydrocortisone.

6. Pharmaceutical composition according to claim 5, wherein the composition comprises 1% (w/w) hydrocortisone.

7. Pharmaceutical composition according to claim 1, wherein the dosage form of the composition is selected from the group consisting of creams, ointments, lotions, pastes, gels and emulsions.

8. Pharmaceutical composition according to claim 7, wherein the composition is in the cream from.

## Patentansprüche

1. Phasenstabile pharmazeutische topische Zusammensetzung, umfassend eine therapeutisch wirksame Menge einer antiviralen Substanz, die Acyclovir ist, und eine therapeutisch wirksame Menge eines entzündungshemmenden Glucocorticoids, das Hydrocortison ist, wobei die Zusammensetzung 10,0-15,0 % (w/w) Cetostearylalkohol und 7,5-12,5 % (w/w) Isopropylmyristat.

2. Pharmazeutische Zusammensetzung nach Anspruch 1, wobei die Zusammensetzung 20 % (w/w) Propylenglykol enthält.

3. Pharmazeutische Zusammensetzung nach Anspruch 1, wobei die Zusammensetzung 0,1-10% (w/w) Acyclovir umfasst.

4. Pharmazeutische Zusammensetzung nach Anspruch 3, wobei die Zusammensetzung 5% (w/w) Acyclovir enthält.

5. Pharmazeutische Zusammensetzung nach Anspruch 1, wobei die Zusammensetzung 0,005-3% (w/w) Hydrocortison umfasst.

6. Pharmazeutische Zusammensetzung nach Anspruch 5, wobei die Zusammensetzung 1 % (w/w) Hydrocortison enthält.

7. Pharmazeutische Zusammensetzung nach Anspruch 1, wobei die Darreichungsform der Zusammensetzung ausgewählt ist aus der Gruppe bestehend aus Cremes, Salben, Lotionen, Pasten, Gelen und Emulsionen.

8. Pharmazeutische Zusammensetzung nach Anspruch 7, wobei die Zusammensetzung in Cremeform vorliegt.

## Revendications

1. Composition topique pharmaceutique à phase stable comprenant une quantité thérapeutiquement efficace d'une substance antivirale qui est l'acyclovir et une quantité thérapeutiquement efficace d'un glucocorticoïde anti-inflammatoire qui est l'hydrocortisone, dans laquelle la composition comprend 10,0 à 15,0 % (p/p) d'alcool cétostéarylique et 7,5-12,5 % (p/p) de myristate d'isopropyle.

2. Composition pharmaceutique selon la revendication 1, dans laquelle la composition comprend 20 % (p/p) de propylèneglycol.

3. Composition pharmaceutique selon la revendication 1, dans laquelle la composition comprend 0,1 à 10 % (p/p) d'acyclovir.

4. Composition pharmaceutique selon la revendication 3, dans laquelle la composition comprend 5 % (p/p) d'acyclovir.

5. Composition pharmaceutique selon la revendication 1, dans laquelle la composition comprend 0,005 à 3 % (p/p) d'hydrocortisone.

6. Composition pharmaceutique selon la revendication 5, dans laquelle la composition comprend 1 % (p/p) d'hydrocortisone.

7. Composition pharmaceutique selon l'une des revendications 1 à 6, **caractérisée en ce que** la forme galénique de la composition est choisie dans le groupe constitué par les crèmes, les pommades, les lotions, les pâtes, les gels et les émulsions.

8. Composition pharmaceutique selon la revendication 7, dans laquelle la composition est sous forme de crème.
